# EUROPEAN PATENT APPLICATION

(11) **EP 4 755 319 A1**
(43) Date of publication of application: **10.06.2026**
(21) Application number: 24217454.8
(22) Date of filing: 04.12.2024
(51) Int. Cl.: A61B 17/00

(54) **A DEVICE FOR SEALING A DEFECT IN A TISSUE OF AN INTERNAL ORGAN OF A PATIENT**

(71) Applicant: CardioCare Spolka z ograniczona odpowiedzialnoscia Spolka komandytowa, 31-159 Krakow (PL)
(72) Inventor: Zalewski, Jaroslaw, 32-087 Zielonki (PL); Schwarz, Tomasz, 31-635 Krakow (PL); Wojtas, Mariusz, 34-600 Limanowa (PL)
(74) Representative: Patpol Kancelaria Patentowa Sp. z o.o.

(57) **Abstract**

The invention regards a device for sealing a defect (9) in the tissue of an internal organ of a patient, having a scaffold comprising longitudinal elastic supports (1) in a radial arrangement similar to an inverted umbrella structure. The invention is characterized in that the device comprises a delivery guidewire (3) and a controlling guidewire (4), wherein the supports (1) are loops which are connected at their distal ends to the delivery guidewire (3) and at their proximal ends to the controlling guidewire (4), and have a peak inflection point, and at the outer surface of the supports (1) an elastic membrane (2) is stretched in the shape of an inverted umbrella cap.

## Description

The subject of the invention is device for sealing a defect in the tissue of an internal organ of a patient having a scaffold containing longitudinal elastic supports in a radial arrangement similar to an inverted umbrella structure. The invention is particularly suitable for the temporary or permanent sealing of a defect in the wall of the left ventricle, and maintaining this sealing for the time necessary for the defect healing, but is not limited to this application.

Left ventricular free wall rupture (FWR) or ventricular septal rupture (VSR) are rare, but associated with a very high risk of death, mechanical complications of myocardial infarction (MI). These situations can also occur independently of myocardial infarction, e.g. as a result of trauma. Before the era of fibrinolytic reperfusion, VSR occurred in 1-3% of patients with MI and FWR in 2-6% of MI patients. The in-hospital mortality rate in patients with VSR was 45% in the absence of shock symptoms and 90% when these symptoms were present, whereas in patients with FWR treated in hospital it was approximately 50%. In the era of fibrinolytic reperfusion, the incidence of VSR and FWR decreased significantly to 0,2-0,3%. In the era of mechanical reperfusion by primary coronary angioplasty, the incidence of these complications is even lower. The peak incidence of FWR and VSR in the era without reperfusion occurred 5-7 days after MI onset. The muscle adjacent to the site of the rupture is usually thinned and fragile. In patients treated with thrombolytic therapy, the median time from chest pain onset to FWR or VSR was 24-30 hours in the GUSTO-I trial and 16-24 hours in the SHOCK trial.

The size of VSR varies from a few millimetres to several centimetres. Morphologically, VSR is classified as simple or complex. A simple perforation has a discrete channel at the same level at both sides of the perforation. In turn, a complex rupture is characterized by a large intramural hematoma and an irregularly shaped channel penetrating the necrotic tissue. Perforations in the septum in patients with anterior wall infarction are usually located near the apex and are generally simple. However, in patients with inferior wall myocardial infarction, VSR is often complex and involves the basal segment of the inferior-posterior part of the septum. Sometimes VSR is accompanied by free wall rupture or tearing of the papillary muscle. Septal perforation causes a shunt from left to right ventricle, with volumetric overload of the right ventricle, increased pulmonary blood flow, and secondary left atrial and left ventricular volumetric overload. As left ventricular systolic function declines and systemic output decreases, compensatory constriction of the systemic vascular bed appears with an increase in systemic vascular resistance, which in turn additionally increases the size of the shunt between the left and right ventricle. The shunt size depends on the size of the perforation, pulmonary vascular resistance, systemic vascular resistance, and the left and right ventricle function. As left ventricular function begins to deteriorate and systolic pressure decreases, left-to-right shunt and its fraction decrease.

In turn, FWR usually results in pericardial tamponade, electromechanical dissociation, and sudden death. In some cases, thrombus adjacent to the place of rupture may occlude the pericardial leak and lead to the formation of a pseudoaneurysm. Based on pathological criteria, FWR can be divided into type I with sudden rupture of a myocardial cleft, type II with an erosion site in the infarct zone showing progressive deterioration of the tear, and type III rupture associated with early left ventricular aneurysm formation. Clinically, depending on the size of FWR and the dynamics of pericardial haemorrhage, FWR can be divided into oozing type and blowout type.

Surgical closure of post-infarction VSR and FWR is the treatment of choice (class I recommendation, level of evidence C). Without this therapy, 90% of patients with VSR and 100% with FWR die within 2 months. In both cases, cardiac surgery may be considered under the protection of intra-aortic counterpulsation and/or left ventricular support using an extracorporeal membrane oxygenation (ECMO) device. Despite significant progress in surgical techniques, mortality in both complications remains very high and, depending on the initial clinical condition, ranges between 10 and 90% in the case of VSR and between 30-40% in the case of FWR.

Koh et al. presented the results of treatment of 25 patients with peri-infarct VSR between years 1997 and 2008. The overall in-hospital mortality rate in the study group was 44% and was significantly higher in the conservatively treated group as compared to the surgery. The mortality rate was higher among patients with infero-basal VSR than with antero-apical VSR.

To date, it has not been clearly established at what timepoint from the MI onset a surgery should be performed. There are arguments for immediate surgical closure of the VSR, regardless of the patient's hemodynamic status, to avoid further worsening of shock symptoms. The argument for early closure of the VSR is the fact that the septal branches of the coronary arteries, subjected to the shear forces of the flowing blood, as well as the process of demarcation of necrotic changes, favour the expansion of the VSR, which may cause hemodynamic decompensation. Many surgeons, on the other hand, believe that the surgery should be postponed for 3-4 or even 6 weeks until scar formation in the tissues surrounding the VSR is completed. It allows for more secure suturing of the edges of the defect and prevents its tearing. However, it should be borne in mind that a period of 3-6 weeks for a patient with features of a significant left-right leak is a very devastating and risky challenge. Hence, in selected patients, the use of less invasive percutaneous methods may be considered to exclude or at least partially reduce the size of the VSR, and thus the right ventricular shunt.

In most cases, acute FWR is associated with sudden death. The dynamic nature of the symptoms makes it difficult to effectively treat even acute FWR diagnosed in hospital. Subacute ruptures offer a chance for successful treatment if diagnosed and immediately transferred to the centre with a cardiac surgery. The gold standard in the treatment of FWR is cardiac surgery to decompress the tamponade and repair the rupture. In cases of rapidly increasing symptoms, the procedure should be accompanied by pericardiocentesis. The largest meta-analysis to date by Matteucci et al. included 363 patients with FWR treated surgically. The authors demonstrated a two-fold lower operative risk in patients with an oozing type of rupture as compared with a blowout type of rupture. The risk of death was 40% lower in patients with FWR repaired with the sutureless technique as compared with those who were sutured. Sutureless surgical sealing of the FWR can be performed using a collagen sponge or a pericardial patch fixated to the epicardium with glue to cover the MI zone. In contrast, the suture technique is defined as the FWR repair with the use of sutures to close the myocardial tear or secure the epicardial patch. Oozing perforations can be sutured, patched, or closed with tissue glue. In surgical survivors, sutured FWRs should be monitored for early detection of leaks or aneurysm formation.

Endovascular VSR procedures are usually performed under general anaesthesia and the device is delivered through a long sheath inserted via arterial and venous femoral access. Most systems for VSR closure work in a similar manner. They usually consist of the same basic components, such as a balloon to measure the size of the perforation, the implant itself, and the system used to deliver it. The implant consists of two foldable discs or umbrellas of different sizes, made of polyester stretched over a metal mesh and separated by a narrow waist or strait, the width of which varies depending on the indication. The delivery system consists of a percutaneously inserted sheath, a guidewire, and a polyurethane catheter of varying length, size, and curvature to optimize the procedure. In the first stage, a diagnostic catheter is placed in the left ventricle and then a guidewire is passed through the VSR to the pulmonary artery where the guidewire is captured and externalized to the femoral vein, creating a continuous arteriovenous loop. The size of the perforation is measured by transesophageal echocardiography and fluoroscopy using a calibrating balloon, and its location is determined by echocardiography. Contraindications to percutaneous VSR closure include perforation size greater than 35 mm, VSR location near the apex of the heart, lack of sufficient edge enabling occluder placement, and location near the mitral valve, tricuspid valve, and/or aortic valve. The closure device threaded onto the guidewire is introduced via the arteriovenous loop created into the area of interest in the ventricular septum, and the device is implanted there.

Sometimes a small residual leak is observed immediately after implantation, but this usually resolves in the following weeks or months. After the occluder is implanted, patients are treated with anticoagulants, usually aspirin, for approximately six months while the occluder heals. Over time, the endocardial tissues and scar tissue fuse with the implant, securing its position permanently and often eliminating any residual leaks. To date, the optimal time for closure of peri-infarct VSRs has not been established. Currently, various devices are used for peri-infarct VSR closure, including the ASD Amplatzer, VSD Amplatzer, or CardioSEAL.

Percutaneous intrapericardial FWR therapy by injection of fibrin glue is an alternative therapy to FWR surgery, especially in the case of the oozing type of FWR. Hattori et al. showed that fibrin glue introduced into the pericardium formed a stable fibrin layer within 1 day, whereas the glue degraded within a week. In turn, Murata et al. in autopsy studies after injection of fibrin glue did not find any inflammatory adhesion of the epicardium to the pericardium. Both studies indicate that fibrin glue is biocompatible and biodegradable.

The available endovascular techniques used for VSR closure have several significant limitations. Rigid sets used to deliver the occluder to the septum may cause the detachment of fragments of necrotic tissue and lead to an increase of the defect size. The implanted occluder, anchored to the fragile septal tissues not infrequently dislocates into the right ventricle, most often requiring cardiac surgery. In addition, the available sizes of occluders may not be sufficient to close large defects, and residual left-to-right leak is one of the most common problems after this type of treatment. Thiele et al. presented the results of treatment of peri-infarct VSR using Amplatzer occluders for atrial or ventricular septal defects. Between years 2003-2009, 29 patients underwent this procedure, 16 of whom had symptoms of cardiogenic shock. Failed occluder implantation in 2 patients with shock resulted in their death by day 30 of follow-up. In turn, of the remaining 14, despite procedural success, 12 (86%) died within a month. On the contrary, in the group of patients without symptoms of shock, the procedure was unsuccessful in 2, and 4 of the 11 successfully treated patients died in 30-day observation.

Endovascular techniques, being less invasive, hold great hope for improving the results of treatment of peri-infarct VSR and FWR. First of all, they can help in the early stabilization of patients with VSR in the course of MI, especially in patients with symptoms of cardiogenic shock, preventing the organ consequences of hypoperfusion. Left ventricular support systems such as ECMO, Impella or TandemHeart, which have not been tested in controlled studies in patients with mechanical complications of MI, are being introduced more and more commonly and with increasingly better results in the treatment of cardiogenic shock. Single case reports or case series indicate that in some clinical situations they may be a valuable adjunct to surgical therapy; however, their use is associated with the risk of widening the zone of left ventricular damage in the case of the Impella or TandemHeart pump or vascular complications in the case of ECMO.

In the case of patients with mechanical complications of MI such as VSR or FWR, the concept of personalized therapy is promoted, which requires understanding the specificity of the disease of each individual patient, recognizing the complexity of their clinical condition with the entire spectrum of accompanying circumstances, and finally selecting the most effective possible therapy. Currently, the specificity of each patient with shock during VSR or FWR is determined on the basis of laboratory tests, echocardiography and hemodynamic measurements. Currently it is obvious that the available therapeutic techniques do not meet the hopes placed in them, thus the new therapies for rare mechanical complications of MI need to be developed.

Rupture and bleeding from the reproductive organs, including the vagina or uterus, affect women of reproductive age and most often during pregnancy. Less frequently, rupture of these organs occurs as a result of trauma. Risk factors for vaginal tearing and bleeding include first intercourse, obstetrical and/or gynaecological history, or a stressful position during intercourse, but a vaginal rupture always require gynaecological intervention.

Uterine rupture is a rare, life-threatening obstetric condition that involves a tear in the wall of the uterus, including the serosa. The incidence of uterine rupture in hospitals ranges from 1 in 100-500 deliveries in developing countries to 1 in 3000-5000 in developed countries. Uterine scarring is the most commonly reported cause of uterine rupture in any trimester, followed by placenta accreta, multiparity, status post post-cesarean section, and unreasonable administration of oxytocin and prostaglandin. In developed countries, the rate of uterine rupture after cesarean section has declined even further, to 1 in 2000 from a global average of 1 in 100. The occurrence of scarless uterine rupture is extremely rare, with estimates ranging from 1 in 8000 to 1 in 15000. Uterine rupture may be accompanied by the use of misoprostol for second trimester abortion. The risk of rupture is then less than 0,35% in a scarred uterus and 0,04% in a non-scarred uterus. Multiparity is another predisposing factor to uterine rupture, as repeated pregnancies lead to thinning of the uterine muscle. The maternal mortality rate due to uterine rupture is 0,2% in developed countries, while in poor countries it can reach up to 30%, mainly due to lack of appropriate care. The risk of uterine rupture leading to hysterectomy ranges from 14 to 33%. Sometimes clinical diagnosis can be difficult because uterine rupture in the first or second trimester of pregnancy is extremely rare and varies in clinical presentation and course. However, uterine rupture should always be considered before considering any other non-gynaecological cause in any pregnant woman who has severe abdominal discomfort, regardless of gestational age. Other common symptoms of uterine rupture include vaginal bleeding, maternal tachycardia, uterine hypotension and/or atony, and abdominal tenderness.

Genitourinary injuries occur in approximately 10% of all abdominal and pelvic injuries, while bladder rupture accounts for only 1,6% of these cases. Due to the structural protection by the bony pelvis, bladder injury is rare and usually associated with high-intensity trauma. Bladder rupture can be classified as extraperitoneal or intraperitoneal. Retroperitoneal ruptures are more common and are usually the result of a forceful blow to the anterior bladder. Ruptures usually result from intravesical pressure following a blow to the abdominopelvic region, which causes rupture of one of the weaker points of the bladder, such as the dome. The clinical presentation of patients with bladder injury may vary depending on the severity of the injury, but most patients report suprapubic pain, gross hematuria, dysuria. Pelvic fractures are very common in patients with bladder injury. Pelvic fractures have been found to be associated with increased morbidity and mortality in patients with bladder injury, and the identification of a pelvic fracture should always raise clinical suspicion to evaluate for a genitourinary injury. Although bladder rupture is rare, it is associated with significant patient morbidity and a mortality rate of approximately 22%. Urological society practice guidelines recommend cystography for stable patients with hematuria and/or pelvic fracture or any other patient with signs and symptoms suggestive of bladder injury. Guidelines state that bladder drainage is the standard of care for both extra- and intraperitoneal bladder ruptures. In addition, the guidelines also recommend surgical repair of all bladder ruptures to prevent peritonitis following intraperitoneal leakage of bladder contents.

Polish patent application No. 433283 discloses a device for closing a peri-infarct perforation of the interventricular septum of the heart, in the form of a scaffold with an openwork supporting structure of a device for closing a peri-infarct perforation of the interventricular septum of the heart, made of biocompatible, hemocompatible and non-toxic materials, which reflects the shape and size of the interior of the left ventricle of the heart, taking into account the papillary muscles and valves. A sealing material is installed on the scaffold with an openwork supporting structure, covering the tissue defect of the ventricular septum of the heart with a significant margin, thus preventing the blood flow from the left to the right ventricle. The primary goal of this device is to reduce mortality and improve prognosis, including the directly life-threatening mechanical complications of MI. In addition, the device can be used in clinical situations where existing methods are ineffective or even contraindicated. The idea of the device is based on the elastic interaction of the interventricular septum, where the peri-infarct perforation is located, with a scaffold covered with a dressing material stretched in the area adjacent to the place of rupture introduced through a transaortic access. The device is intended to stop uncontrolled shunt from the left to the right ventricle of the heart, further deoxygenation of arterial blood and, finally deterioration of left ventricular contractility. This VSR closure device, covering the left ventricular endocardium, is anchored in the left ventricular outflow tract and elastically deforms with the contracting and relaxing left ventricle.

The device described above, although it solves the problems of the prior art, has imperfections which are eliminated by the subject of the present invention.

The present invention relates to a device for sealing a tissue defect in an internal organ of a patient, having a scaffold comprising longitudinal resilient supports in a radial arrangement similar to an inverted umbrella structure. The device is characterized in that it comprises a delivery guidewire and a controlling guidewire, wherein the supports are loops which are connected to the delivery guidewire at their distal ends and to the controlling guidewire at their proximal ends, and have a peak inflection point, and an elastic membrane is stretched on the outer surface of the supports in the shape of an inverted umbrella cap.

Preferably, the supports have a flattened profile, more preferably they are tapes or flat wires.

Preferably, the supports are arranged at regular circumferential intervals around the guidewires.

Preferably, the device comprises at least four supports.

Preferably, the supports are made of a shape memory material, more preferably of a NiTi alloy, having a width of approximately 0,8 mm and thickness of approximately 0,15 mm.

Preferably, the membrane is reinforced with a flexible strut made of shape memory material running along the entire circumference of the membrane.

More preferably, the strut has a sinusoidal course, even more preferably with a number of periods corresponding to the number of supports, and an amplitude of about 10-50% of the length of the supports, wherein the minimum points of the sinusoid are located at the points of contact of the strut with the supports, near the middle of their course, and the maximum points of the sinusoid are located in the middle of the distance between the supports, slightly below their peak inflection point.

Even more preferably, the strut is integral with the membrane.

Also preferably, the membrane is made of PCL.

More preferably, the strut is made of a NiTi alloy.

Preferably, the delivery guidewire and the controlling guidewire are slidably connected by a proximal guide sleeve, the guide sleeve being fixedly attached to the controlling guidewire and slidably surrounding the delivery guidewire, and the proximal ends of the supports being fixedly connected to the controlling guidewire via the guide sleeve.

Preferably, the distal ends of the supports are connected to the delivery guidewire by means of a distal fixed sleeve.

Preferably, the delivery guidewire and the controlling guidewire are made of a NiTi alloy, and more preferably comprise a round wire having a diameter of about 0,5 mm.

More preferably, the guide sleeve and the fixed sleeve are made of silver, surgical steel or titanium.

Preferably, the device has spikes arranged circumferentially at the distal end of the device, at the outside surface of the membrane, in the part surrounding the point of connection of the supports.

Preferably, the device has 3-5 spikes, even more preferably 2-3 mm high.

Preferably, the membrane is attached to the supports in a non-permanent manner, more preferably by means of resorbable surgical threads, enabling the membrane to be left in the patient's body and the remaining part of the device to be removed.

More preferably, the membrane is covered with a cell proliferation accelerating layer and the supports are covered with an antithrombotic layer.

Preferably, at least one support is provided with a radiological marker.

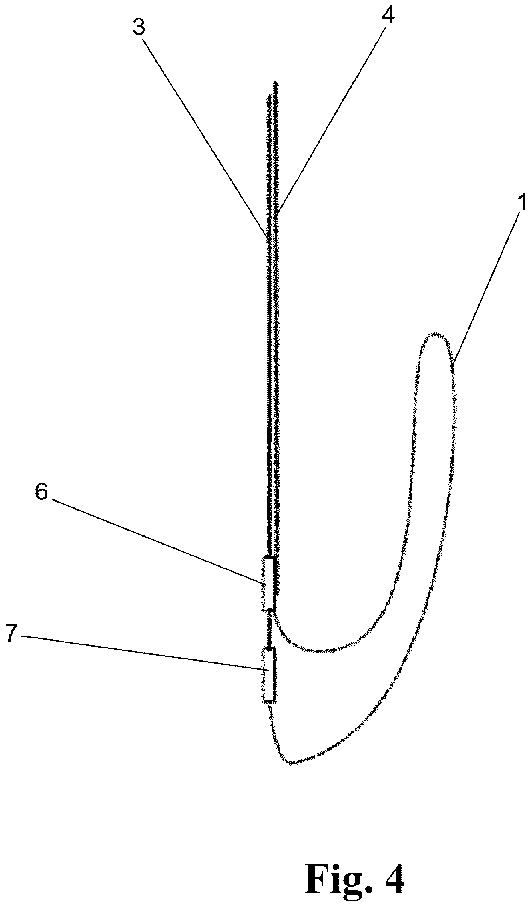

The subject of the invention is explained in the embodiments in the drawings, in which FIG. 1 shows a schematic view of the structure of the device according to the invention, in an embodiment, FIG. 2 shows the principle of placement and position of the device in the patient's organ with a wall defect, FIG. 3 shows a possible method of delivering the device to an organ, showing the device in a folded configuration, placed in a catheter (3a), and in an unfolded configuration, respectively, before insertion into the catheter or after its release (3b), and the functioning of the device after placement in the patient's organ - here in the heart ventricle in diastole (c) and systole (d), and FIG. 4 shows in more detail, in an example embodiment, the arrangement of the guidewires and the method of connecting them.

The figures show that the device for sealing a defect 9 in the tissue of an internal organ of a patient has a scaffold comprising longitudinal elastic supports 1 in a radial arrangement similar to an inverted umbrella structure, wherein the device comprises a delivery guidewire 3 and a controlling guidewire 4, and the supports 1 are loops which are connected at their distal ends to the delivery guidewire 3 and at their proximal ends to the controlling guidewire 4, and have an peak inflection point, while on the outer surface of the supports 1 an elastic membrane 2 is stretched in the shape of an inverted umbrella cap.

Due to its structure, i.e. the fact that the supports 1 and the membrane 2 are flexible, the device can be folded and placed together with the delivery and controlling guidewires 3 and 4 in the delivery catheter 11.

Of course, due to the purpose of the device, all elements of the device are made of biocompatible, hemocompatible and non-toxic materials, with mechanical characteristics that allow them to adapt to the inner surface of the patient's internal organ in which the existing defect 9 is to be sealed. In the case of using a device to seal a defect 9 in the heart ventricle, the supports 1 must have mechanical properties associated with high elasticity and, preferably, shape memory, thanks to which they remain in constant contact with the endocardial surface both during the systole and diastole phases of the heart ventricle. At the same time, the elements must be flexible enough so that the resistance to the contracting ventricle does not impair its mechanical work and contraction capacity, considering the weakened strength of the myocardium in the area affected by pathological changes that may be the cause of the defect 9. An important factor in the usefulness of the material is also its resistance to repeated compression, which allows the structure to remain in the heart ventricle for the time necessary for the defect healing 9 without mechanical damage to the load-bearing elements of the structure. The expected time required for healing of the ventricle wall 10 is typically 3 to 6 weeks, which equates to 1,8 to 6 million ventricular cycles.

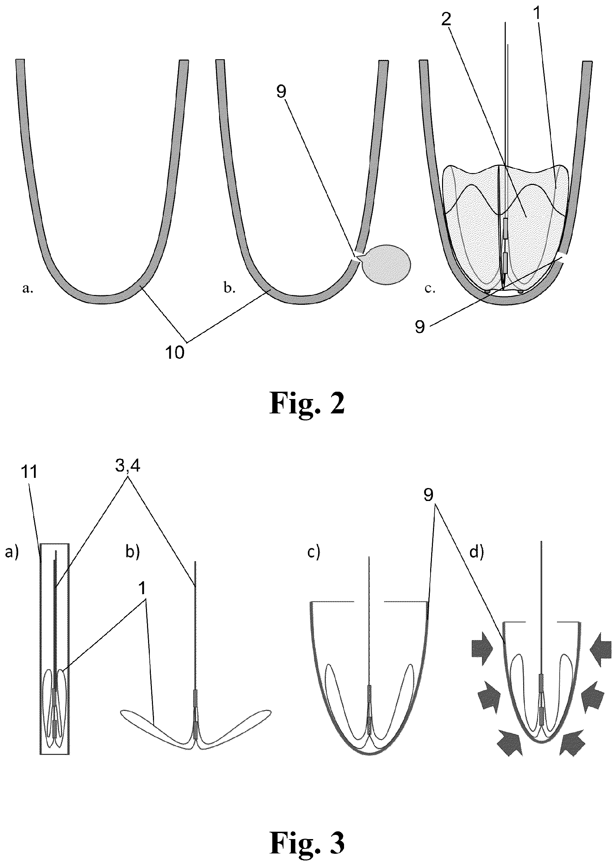

When used in the heart ventricle, the spatial geometry of the device design assumes that its apex point is fitted to the apical part of the left ventricle. When the patient is in a vertical position, this point is the lower end of the device and all supports 1 are connected to it. From this point the supports 1 extend from the delivery guidewire 3 along the surface of the ventricular wall 10 upwards towards the base of the heart. The length of the supports 1 (arms) of the device stretched in the ventricle is selected in a way that allows for covering the defect 9, but does not pose a risk of blocking or damaging the internal structures of the ventricle responsible for its proper function. First of all, the supports 1 of the device bypass the papillary muscles and end below the course of the chordae tendineae responsible for the tightness of the mitral valve. The device supports 1 are connected at their distal end to the delivery guidewire 3 and, at a level below the attachment of the chordae tendineae to the papillary muscles, are bent towards the centre of the ventricle and reconnected to the controlling guidewire 4. Both guidewires 3,4 are led outside the ventricle. The delivery guidewire 3 is used to introduce the device by means of the delivery catheter 11 (FIG. 3a) to the target site, i.e. in this example to the periapical region of the left ventricle. The controlling guidewire 4 allows the operator to vary the spatial geometry of the device from the moment of its insertion into the catheter 11 when the device is assembled, to the moment when the device achieves its final working shape within the ventricle, fitted and stabilized in its target location.

The structure, when folded in catheter 11, is longer but significantly narrower (FIG. 3a). FIG. 3c shows that when the device is deployed within the heart ventricle, thanks to the shape memory effect stored in the material, the device takes on the proper shape of an inverted umbrella, adapting to the periapical part of the ventricle, while the supports 1 are arranged along the ventricle walls, in its long axis, resting on them up to the point of bending. The operation of the controlling guidewire 4 when the position of the delivery guidewire 3 is locked makes it possible to increase or decrease the distance between the base and the top of the device, which results in an increase or decrease in the degree of spreading of the supports 1 of the structure and an increase or decrease in the tension of the membrane 2 stretched on the supports, respectively. The optional possibility of locking the position of the controlling guidewire 4 in relation to the delivery guidewire 3 fixes the degree of distribution and tension of the structure supports in an arrangement optimal for the size of the heart ventricle and its contraction force.

An elastic membrane 2 is installed on the supports 1 of the device along the length of their contact with the surface of the organ, which is intended to fulfil the functional assumptions of the device, i.e. stopping the outflow of blood or other body fluid through the defect outside the cavity of the patient's organ (see FIG. 2, which schematically shows a cross-section of a healthy organ, FIG. 2a, organ with defect 9, FIG. 2b, and the organ with the defect 9 closed by means of the device according to the invention). Correct placement of the supporting structure of the device inside the organ ensures tight adhesion of the membrane 2 to its inner surface, while the pressure system inside the organ provides an additional stabilizing and pressing effect. When the device is placed inside the heart ventricle, during diastole and opening of the mitral valve, the pressure of blood flowing into the left ventricle gently presses the membrane 2 against the endocardial surface, ensuring its tight fit and seal. During ventricular contraction, the increasing blood pressure constitutes an additional element pressing on the membrane 2, while the decreasing volume of the ventricle during the ejection of blood into the aorta maintains the pressure and thus the force pressing the membrane 2 against the endocardium. Compared to the prior art, the membrane 2 is tight around its entire circumference, which means that the device will effectively close defects 9 at any location, provided that the defect 9 is located under the membrane. In the case of the prior art mesh dressing, it is necessary to position the device very precisely so that the dressing is placed on the defect. This made the procedure significantly complicated and prolonged and also limited its effectiveness. Moreover, during the research it turned out that it is difficult to find a method for introducing and, above all, properly unfolding the mesh structure. It would probably be necessary to use a balloon (similarly to stents), but the ability to fold the device for removal would then be problematic. This would certainly be impossible using the percutaneous method. The present invention also solves this problem. The prior art mesh additionally generated a large contact area between the structure and the interior of the organ wall 10, and over a large area there was contact of the part of the structural mesh without the dressing, which could result in ingrowth of the structure, which should be avoided. In the present invention, the supports do not contact the interior of the organ wall 10 at all, since they are isolated from it by a membrane along its entire perimeter. Moreover, the appropriate working together of the mesh with the organ is more difficult to achieve compared to the operation of the device according to the present invention, especially when used in the heart ventricle, in the systolic phase, when the supports yield to the pressing wall and the mesh tends to undulate, but also in diastole, when the supports follow the dilatating ventricular wall and the mesh, undulating in systole, may not keep up.

The material for membrane 2 is, similarly to the material of the remaining elements of the device, biocompatible, hemocompatible and non-toxic. From the point of view of mechanical properties, it is made of a material with high elasticity, which allows it to fit inside the organ and the geometry of the supporting structure (supports 1) of the device, as well as tightness ensuring effective blocking of the flow of fluid through the defect in the tissue of the organ wall 10. The preferred material for membrane 2 is PCL.

An important aspect of the device is also the cross-sectional profile of the supports 1, ensuring their appropriate operation inside the organ (in the case of the heart ventricle - towards the inside of the ventricle during systole and towards the outside during diastole), without generating lateral deflections that could destabilize the position of the device and change the spatial arrangement of the structure. The optimal option seems to be a flattened profile of the supports 1 (tape, flat wire), with the wider surface directed towards the wall 10 of the organ, which will prevent the occurrence of the above-mentioned lateral deflections, however the shape of the supports 1 is not limited to this, and any other shape can be used that will provide real advantages in a given application. The best material for supports 1 is NiTi alloy (nitinol). For use inside the heart ventricle, the optimal dimensions are approximately 0,8 mm wide and approximately 0,15 mm thick (considering manufacturing variations).

In a preferred embodiment, the supports 1 are arranged at regular circumferential intervals around the guidewires 3, 4 and it is the best if there are at least four of them. This example is preferred for use in the heart ventricle. In certain embodiments, however, the supports may be arranged irregularly and their number may vary, for example in smaller organs there may be 3 supports 1, but in principle it is a question of a balance between the number of supports 4 providing sufficient support and pressure to secure the defect 9 in the organ, while at the same time not being too large (the more supports 1 there are, the larger the volume of the composite device, which requires the use of a larger and therefore thicker catheter 11 to introduce the device into the organ; this becomes problematic at some point, as the capacity of the blood vessels has its limits). The supports can also be arranged irregularly to accommodate organs with less regular shapes.

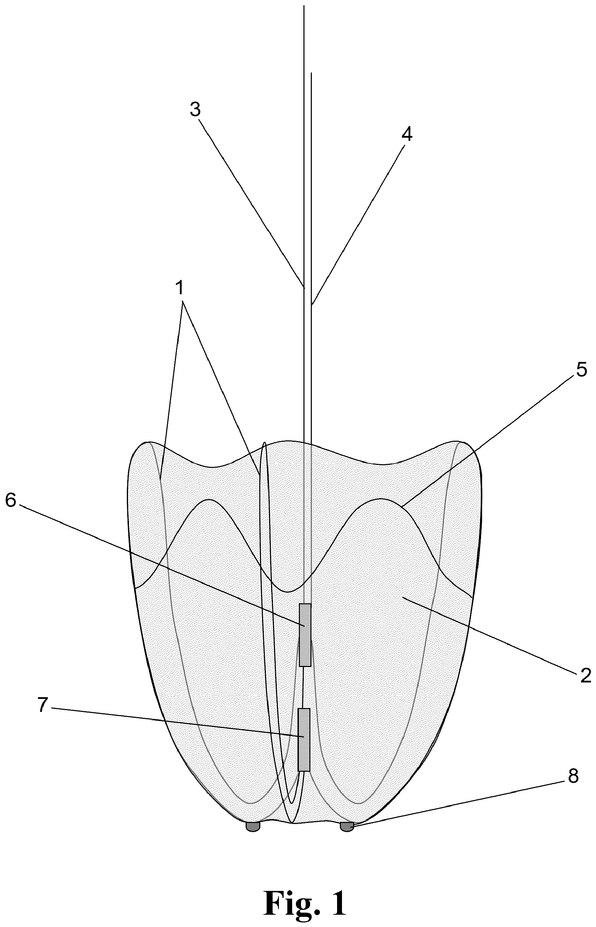

In the preferred embodiment, shown in FIG. 1-2, the membrane 2 is reinforced with an elastic strut 5 made of a shape memory material (preferably a NiTi alloy, i.e. nitinol), running along the entire circumference of the membrane 2. Preferably, the strut 5 has a sinusoidal course, preferably with a number of periods corresponding to the number of supports 1 and an amplitude of about 10-50% of the length of the supports 1, wherein the minimum points of the sinusoid are located at the places of contact of the strut 5 with the supports 1, near the middle of their course, and the maximum points of the sinusoid are located in the middle of the distance between the supports 1, slightly below the point of their peak inflection.

The purpose of the strut 5 is to improve the pressure of the membrane 2 against the inner surface of the organ and to prevent fluid from entering between the membrane 2 and the inner surface of the organ. The sinusoidal shape of the strut 5 provides flexibility that allows the entire device (support structure including the supports 1 together with the installed membrane 2) to be folded and fitted into the delivery catheter 11. Additionally, it also makes it possible to reduce the circumferential length of the strut 5, e.g. during ventricular contraction, preventing the development of excessive resistance that could lead to blockage of the ventricular contractile function. The shape memory enables the strut 5 to unfold and assume the proper shape upon withdrawal of the device from the delivery catheter 11 within the heart ventricle, and also to follow the heart wall 10 during diastole to improve compression of the membrane 2 against the endocardium. It is optimal if the strut 5 is an integral part of the membrane 2 and can be hidden between its layers for more complete integration. Therefore, the strut 5 is not intended to be an element of the load-bearing structure, but only an auxiliary element of the membrane 2, and is attached to the supports 1 together with the membrane 2 but not independently on it.

In the embodiment shown in the figures, the delivery guidewire 3 and the controlling guidewire 4 are slidably connected by means of a proximal guide sleeve 6, wherein the guide sleeve 6 is fixedly attached to the controlling guidewire 4 and slidably surrounds the delivery guidewire 3, and the proximal ends of the supports 1 are fixedly connected to the controlling guidewire 4 via the guide sleeve 6. It is advantageous if the guide sleeve 6 is a clamping sleeve and the distal ends of the supports 1 are connected to the delivery guidewire 3 by means of a distal fixed sleeve 7. The skilled person will appreciate that the guidewires do not have to be connected to each other, but their connection improves the stability of the device, the simplicity of insertion of the device into the organ, and stabilizes the movement of the controlling guidewire 4 longitudinally relative to the delivery guidewire 3, preventing side or rotary movement. In a preferred embodiment, the delivery guidewire 3 and the controlling guidewire 4 are made of a NiTi alloy, and preferably consist of a wire with a round cross-section and have a diameter of about 0,5 mm (within manufacturing tolerances). The guide sleeve 6 and the fixed sleeve 7 may be made of e.g. silver, surgical steel or titanium, and the inner surface of the guide sleeve 6 may be covered with a material facilitating its sliding on the delivery guidewire 3, preferably graphite, Teflon or another material with similar properties.

In the embodiment shown in FIG. 1-2 the device has spikes 8 arranged circumferentially at the distal end of the device, outside the membrane 2, in the part surrounding the connection point of the supports 1. The spikes 8, directed towards the wall 10 of the organ, are an additional element stabilizing the position of the device in the organ. It is preferable to have 3-5 spikes 8, preferably 2-3 mm high.

In a preferred embodiment, the membrane 2 is attached to the supports 1 in a non-permanent manner, preferably by means of resorbable surgical threads, enabling the membrane 2 to be left in the patient's body and the remaining part of the device to be removed. In this example, it is good if the membrane 2 is covered with a layer accelerating cell proliferation and the supports 1 are covered with an antithrombotic layer.

In an embodiment, at least one support 1 is provided with a radiological marker to enable visualization of the device.

As discussed with respect to the above embodiments, the method of installing the membrane 2 on the supports 1 of the device assumes design variants (permanent or non-permanent connection of the membrane 2 to the supports 1), with three possible functional variants.
1. Permanent installation using non-resorbable materials that are resistant to the conditions in the patient's body allows the device to remain in one piece during the healing period of the tissues in the defect area, and then the device is removed as the whole (membrane and supports) during a second procedure aimed at final closure of the defect using a cardiac surgical method (suturing) or percutaneously (using an occluder). In this situation, the supports and the membrane must be covered with an anticoagulant layer to prevent clotting and ingrowth of the membrane into the healing tissue.
2. Permanent installation using non-resorbable materials resistant to the conditions in the left ventricle of the heart, with the membrane covered with substances stimulating tissue integration of the membrane with the endocardium and leaving the entire structure in the heart forever.
3. Non-permanent installation using resorbable materials, enabling tissue integration of the membrane with the organ by stimulating endogenous mechanisms, while simultaneously releasing the membrane from the supporting structure of the device. Then, after removing the supporting structure, the membrane will remain in the organ, integrated with the wall. In this situation, only the supports need to be covered with an anticoagulant layer to protect against clotting, while the membrane should be covered with a layer that accelerates cell proliferation.

In the embodiment, three possible methods of introducing the device into the left ventricle of the heart are assumed:
i. a more invasive surgical approach using a small access right lateral thoracotomy, the closure device will be implanted via a transaortic approach using cardiopulmonary bypass;
ii. less invasive percutaneous method, by transarterial or transvenous route.

The surgical method assumes that in patients who are intubated, breathing with a ventilator, with an introduced central venous line, arterial line and electrocardiogram, under general anaesthesia, the heart will be exposed via a right thoracotomy. Appropriate cannulas will be inserted into the ascending aorta and right atrium via the right jugular vein. An extracorporeal circulation will be connected and once full flow is achieved, the ascending aorta will be clamped and cardiac arrest will be induced. Under visual guidance, a system with a perforation closure device will be delivered through the ascending aorta and then through the aortic valve into the left ventricle. Under angioscopy guidance, the device will be introduced into the left ventricular cavity and the endocardial surface will be covered with the device scaffold from the apex to the base of the papillary muscles. Depending on the patient's clinical condition, between 4 and 6 weeks from the beginning of the convalescence period, under general anaesthesia, on cardiopulmonary bypass, via an anterior thoracotomy via the right ventricular approach, the occlusive device will be removed, and the perforation will be sutured or closed with an occluder.

The percutaneous method assumes that in intubated patients, breathing from a ventilator, with an introduced central venous line, arterial line and electrocardiogram, under general anaesthesia, arterial access will be made (from the femoral, basilic or carotid artery), through which, using a delivery catheter, the device will be introduced through the ascending aorta and aortic valve into the interior of the left ventricle, under the control of fluoroscopy and transesophageal or intracardiac echocardiography. After unfolding and stabilizing the device inside the ventricle, it will be additionally secured by sewing of guidewires with external access. This means that after the recovery period, the device can be removed the same way it was inserted, without the need for an invasive surgical procedure. The further procedure will depend on the method of installing the membrane on the device structure. In the case of permanent installation, after the device is removed, a cardiac occluder can be used as a continuation of non-invasive therapy, which will permanently close the tissue defect. In the case of a non-permanent installation, removal of the supporting structure and confirmation of successful closure of the perforation by the ingrown membrane will be the final step of the interventional therapy.

The choice of device design and membrane installation method, and thus the treatment procedure used, will depend on the patient's general condition and the size of the defect. Therefore, it is assumed that both variants of the device will be made available for medical practice and the safest and most effective therapy will be up to the doctor decision.

The third method used is also percutaneous but uses venous access. In intubated patients, breathing from a ventilator, with an introduced central venous line, arterial line and electrocardiogram, under general anaesthesia, venous access (from the jugular vein) will be performed with an entrance to the right atrium of the heart. Using a transseptal needle, a puncture will be made in the interatrial septum, giving access to the left atrium of the heart and, through the mitral valve, to the left ventricle. Through this access, using a delivery catheter, the device will be introduced into the left ventricle under the control of fluoroscopy and transesophageal or intracardiac echocardiography. After unfolding and fixing the device inside the ventricle, it will be additionally secured by sewing of guidewires with external access. The remaining procedures will be identical to those for arterial access. The advantage of this solution is: i. a larger caliber of the venous vessel as compared to the arterial vessel, which allows for the safe use of a larger delivery catheter; ii. increased long-term patient safety, due to a lower risk of occlusion of the vein lumen as compared to the artery, through clotting on the guidewires of the device running inside the blood vessel.

The device, with certain modifications in size and shape, can also be used in organs other than the heart, if they have internal space and a possible route of application. Examples of additional applications of the device may include: i. sealing of bladder tissue defects causing urine flow into the peritoneal cavity, with application through the urethra; ii. sealing of uterine perforation, with application through the vagina; iii. sealing of perforation in certain sections of the gastrointestinal tract causing food flow into the peritoneal cavity, with application through the esophagus (in the case of proximal sections) or through the anus (in the case of distal sections).

Of course, the invention is not limited to the implementation examples described above, and the features indicated in the claims can be combined in any combination appropriate to the particular application of the solution.

## Claims

1. A device for sealing a defect (9) in the tissue of an internal organ of a patient, having a scaffold comprising longitudinal elastic supports (1) in a radial arrangement similar to an inverted umbrella structure, **characterized in that** the device comprises a delivery guidewire (3) and a controlling guidewire (4), wherein the supports (1) are loops which are connected at their distal ends to the delivery guidewire (3) and at their proximal ends to the controlling guidewire (4), and have a peak inflection point, and at the outer surface of the supports (1) an elastic membrane (2) is stretched in the shape of an inverted umbrella cap.

2. The device according to claim 1, **characterized in that** the supports (1) have a flattened profile, preferably they are tapes or flat wires.

3. The device according to claim 1 or 2, **characterized in that** supports (1) are arranged at regular circumferential intervals around the guidewires (3, 4).

4. The device according to one of the claims 1-3, **characterized in that** it contains at least four supports (1).

5. The device according to one of the claims 1-4, **characterized in that** the supports are made of a shape memory material, preferably a NiTi alloy, having a width of approximately 0,8 mm and a thickness of approximately 0,15 mm.

6. The device according to one of the claims 1-5, **characterized in that** the membrane (2) is reinforced with a flexible strut (5) made of shape memory material, running along the entire circumference of the membrane (2).

7. The device according to claim 6, **characterized in that** the strut (5) has a sinusoidal course, preferably with a number of periods corresponding to the number of supports (1) and an amplitude of about 10-50% of the length of the supports (1), wherein the minimum points of the sinusoid are located at the points of contact of the strut (5) with the supports (1), near the middle of their course, and the maximum points of the sinusoid are located in the middle of the distance between the supports (1) slightly below the point of their peak inflection.

8. The device according to claim 6 or 7, **characterized in that** the strut (5) is integral with the membrane (2).

9. The device according to one of the claims 1-8, **characterized in that** the membrane (2) is made of PCL.

10. The device according to one of claims 6-9, **characterized in that** the strut (5) is made of NiTi alloy.

11. The device according to one of the claims 1 - 10, **characterized in that** the delivery guidewire (3) and the controlling guidewire (4) are slidably connected by means of a proximal guide sleeve (6), wherein the guide sleeve (6) is fixedly attached to the controlling guidewire (4) and slidably surrounds the delivery guidewire (3), and the proximal ends of the supports (1) are fixedly connected to the controlling guidewire (4) by means of the guide sleeve (6).

12. The device according to one of the claims 1 - 11, **characterized in that** the distal ends of the supports (1) are connected to the delivery guidewire (3) by means of a distal fixed sleeve (7).

13. The device according to one of the claims 1 - 11, **characterized in that** the delivery guidewire (3) and the controlling guidewire (4) are made of a NiTi alloy and preferably they comprise a round wire with a diameter of about 0,5 mm.

14. The device according to one of the claims 11 - 13, **characterized in that** the guide sleeve (6) and the fixed sleeve (7) are made of silver, surgical steel or titanium, wherein the inner surface of the guide sleeve (6) is coated with a material facilitating sliding of the guide sleeve (6) on the delivery guidewire (3), preferably with graphite, Teflon or another material with similar properties.

15. The device according to one of the claims 1 - 14, **characterized in that** it has spikes (8) arranged circumferentially at the distal end of the device, at the outside surface of the membrane (2), in the part surrounding the connection point of the supports (1).

16. The device according to claim 15, **characterized in that** it has 3-5 spikes (8), preferably 2-3 mm high.

17. The device according to one of the claims 1 - 16, **characterized in that** the membrane (2) is attached to the supports (1) in a non-permanent manner, preferably using resorbable surgical threads, allowing the membrane (2) to be left in the patient's body and the remaining part of the device to be removed.

18. The device according to claim 17, **characterized in that** the membrane (2) is covered with a cell proliferation accelerating layer, and the supports (1) are covered with an antithrombotic layer.

19. The device according to one of the claims 1 - 18, **characterized in that** at least one support (1) is provided with a radiological marker.
